# EUROPÄISCHE PATENTSCHRIFT

(11) **EP 2 160 165 B1**
(45) Veröffentlichungstag und Bekanntmachung des Hinweises auf die Patenterteilung: **14.09.2011**
(21) Anmeldenummer: 07785545.0
(22) Anmeldetag: 23.06.2007
(51) Int. Cl.: A61F 13/00

(54) **MAGNETISIERBARE TAMPON-VORRICHTUNG**
MAGNETIZABLE TAMPON DEVICE
TAMPON MAGNÉTISABLE

(43) Veröffentlichungstag der Anmeldung: 10.03.2010
(73) Patentinhaber: Sulger, Christian, 88453 Erolzheim (DE)
(72) Erfinder: Sulger, Christian, 88453 Erolzheim (DE)
(74) Vertreter: Kiessling, Christian
(86) Internationale Anmeldenummer: PCT/DE2007/001111
(87) Internationale Veröffentlichungsnummer: WO 2009/000220

(56) Entgegenhaltungen:
- WO-A-00/66062
- DE-A1- 3 410 715
- DE-U1-202006 005 424
- GB-A- 1 283 521
- GB-A- 2 291 374
- US-A1- 2004 064 114

## Beschreibung

Die Erfindung betrifft eine Tampon-Vorrichtung zum Einführen in die weibliche Vulva, mit einer Emballage aus saugfähiger Substanz zur Aufnahme von Menstruationsflüssigkeit, wobei in die Emballage eine mit dem Erdmagnetfeld in Wechselwirkung tretende magnetische Substanz integriert ist.

Tampon-Vorrichtungen der eingangs genannten Art werden im Stand der Technik verwendet, um ein Auffangen und kurzzeitiges Speichern von Menstruationsflüssigkeit noch in der Vulva zu ermöglichen, da dadurch die Bewegungsfreiheit einer die Tampon-Vorrichtung nutzenden Person nicht eingeschränkt ist. Die bekannten Vorrichtungen weisen jedoch den Nachteil auf, dass eine gleichzeitige Behandlung von Menstruationsbeschwerden im weiteren Sinne nicht ermöglicht ist.

Aus US 2004/0064114 Al ist ein System zum Detektieren des Feuchtigkeitsgehaltes einer feuchtigkeitsabsorbierenden Materialien bekannt, bei dem ein Sensor ein elektrisches Ausgangssignal liefert, das von der Menge wässriger Flüssigkeit abhängt, die in dem Material enthalten ist.

Aufgabe der Erfindung ist es, eine Tampon-Vorrichtung zu schaffen, mittels derer Menstruationsbeschwerden im weiteren Sinne wie schmerzhafte Muskelverspannungen und Krämpfe im Bereich der Vulva-muskulatur gelindert oder behoben werden können.

Für eine Vorrichtung der eingangs genannten Art wird diese Aufgabe gemäß einem ersten Aspekt der Erfindung dadurch gelöst, dass die magnetische Substanz in Form eines Stabmagneten ausgebildet ist, der in Richtung der Längsachse des zylinderförmigen Körpers orientiert angeordnet ist, der zylinderförmige Körper eine Länge im Bereich von etwa 2,0 cm bis 5,0 cm bei einem Durchmesser von etwa 0,5 cm bis 1,5 cm aufweist, und ein mittels der magnetischen Substanz verursachtes Magnetfeld eine Stärke aufweist, die dem 0,5-fachen bis 2-fachen der Stärke des Erdmagnetfeldes entspricht. Die magnetische Substanz ist dabei geeignet, durch einen im Stand der Technik bekannten Magnetisierungsprozess so magnetisiert zu werden, dass sie permanent ein Magnetfeld vorgegebener Stärke aufweist.

Gemäß einem zweiten Aspekt der Erfindung wird diese Aufgabe für eine Vorrichtung der eingangs genannten Art dadurch gelöst, dass die Emballage im wesentlichen im Form eines zylinderförmigen Körpers mit abgerundeten Stirnflächen ausgebildet ist, wobei die Stromquelle, die magnetische Substanz umhüllende elektrische Spule sowie die Aktivierungseinrichtung im Bereich der Längsachse des zylinderförmigen Körpers vorgesehen sind, und der zylinderförmige Körper dabei eine Länge im Bereich von etwa 2,0 cm bis 5,0 cm bei einem Durchmesser von etwa 0,5 cm bis 1,5 cm aufweist. Die magnetische Substanz ist dabei geeignet, durch Wirkung des von der Spule erzeugten Wechsel-Magnetfeldes den Magnetisierungsgrad zu ändern, wobei ein Magnetfeld mit sich im Lauf der Zeit vorgebbar ändernder Stärke erzeugt wird.

Bevorzugte Ausführungsformen der Erfindung sind Gegenstand der Unteransprüche der jeweiligen unabhängigen Patentansprüche.

Bei der erfindungsgemäßen Tampon-Vorrichtung gemäß erstem Aspekt wird durch das Merkmal, dass die magnetische Substanz in Form eines Stabmagneten ausgebildet ist, der in Richtung der Längsachse des zylinderförmigen Körpers orientiert angeordnet ist, der zylinderförmige Körper eine Länge im Bereich von etwa 2,0 cm bis 5,0 cm bei einem Durchmesser von etwa 0,5 cm bis 1,5 cm aufweist, und ein mittels der magnetischen Substanz verursachtes Magnetfeld eine Stärke aufweist, die dem 0,5-fachen bis 2-fachen der Stärke des Erdmagnetfeldes entspricht, erreicht, dass durch die ständige Bewegung der die Tampon-Vorrichtung tragenden Person eine permanente Orientierungsänderung des durch die magnetische Substanz verursachten Magnetfeldes mit dem Erdmagnetfeld gegeben ist, wobei die Kraft des resultierenden Magnetfeldes bei einer Gleichausrichtung der Summe dieser beiden Felder und bei entgegengesetzter Ausrichtung der Differenz aus dem betreffenden stärkeren Magnetfeld und dem betreffenden schwächeren Magnetfeld entspricht.

In den übrigen Zwischenpositionen beim Tragen der erfindungsgemäßen Tampon-Vorrichtung ist die Kraft des resultierenden Magnetfeldes in einem Zwischenbereich zwischen diesen Extremwerten vorzufinden. Eine durch Bewegung bedingte ständige Orientierungsänderung der Lage der erfindungsgemäßen Tampon-Vorrichtung bewirkt somit eine permanente, in ihrem Ausmaß stochastische Änderung des die magnetische Substanz umhüllenden Gesamtmagnetfeldes. Das so verursachte Wechselfeld ist geeignet, menstruationsbedingte Krämpfe der Vulvamuskulatur sowie Muskelspannungen und Muskelverspannungen zu lösen.

Bei der erfindungsgemäßen Tampon-Vorrichtung gemäß dem zweitem Aspekt wird durch die Merkmalskombination, dass die Emballage im wesentlichen im Form eines zylinderförmigen Körpers mit abgerundeten Stirnflächen ausgebildet ist, wobei die Stromquelle, die magnetische Substanz umhüllende elektrische Spule sowie die Aktivierungseinrichtung im Bereich der Längsachse des zylinderförmigen Körpers vorgesehen sind, und der zylinderförmige Körper dabei eine Länge im Bereich von etwa 2,0 cm bis 5,0 cm bei einem Durchmesser von etwa 0,5 cm bis 1,5 cm aufweist, erreicht, dass ein gemäß der Vorgabe aus der Aktivierungseinrichtung sich definiert im Lauf der Zeit änderndes Magnetfeld erzeugt wird, das direkt auf das umgebende Muskelgewebe einwirkt und dabei die Menstruationsbeschwerden lindert oder sogar vollständig beseitigt. Die Aktivierungseinrichtung kann dabei mittels eines programmierbaren Chips gesteuert sein, oder sie kann eine fest verdrahtete analoge Schaltung beinhalten.

Die erfindungsgemäßen Lösungen weisen beide den Vorteil gegenüber vorbekannten Lösungsansätzen auf, dass keinerlei chemische oder pharmazeutische Substanzen benötigt werden.

Gemäß bevorzugter Ausführungsformen der erfindungsgemäßen Vorrichtung gemäß erstem Aspekt ist vorgesehen, dass die magnetische Substanz von Eisen, oder von Nickel gebildet ist.

Die magnetische Substanz ist dabei vorzugsweise in Form eines Stabmagneten ausgebildet, und ist ebenfalls vorzugsweise im Inneren der Emballage angeordnet ist.

Gemäß einer weiteren bevorzugten Ausführungsform der erfindungsgemäßen Vorrichtung gemäß erstem Aspekt ist vorgesehen, dass die Emballage im wesentlichen im Form eines zylinderförmigen Körpers mit abgerundeten Stirnflächen ausgebildet ist, wobei die magnetische Substanz im Bereich der Längsachse des zylinderförmigen Körpers vorgesehen ist.

Gemäß bevorzugter Ausführungsformen der erfindungsgemäßen Vorrichtung gemäß zweitem Aspekt ist vorgesehen, dass die Aktivierungseinrichtung eine Rechteckspannung oder einen sinusförmigen Spannungsverlauf erzeugt.

Die magnetische Substanz ist auch hier vorzugsweise von Eisen, oder Nickel gebildet und ebenfalls vorzugsweise in Form eines innerhalb der Spule angeordneten Stabmagneten ausgebildet.

Gemäß einer weiteren bevorzugten Ausführungsform der erfindungsgemäßen Vorrichtung gemäß zweitem Aspekt ist vorgesehen, dass die Stromquelle, die die magnetische Substanz umhüllende elektrische Spule sowie die Aktivierungseinrichtung im Inneren der Emballage angeordnet sind.

Auch bei der erfindungsgemäßen Vorrichtung gemäß zweitem Aspekt ist die magnetische Substanz in Form eines Stabmagneten ausgebildet, der in Richtung der Längsachse des zylinderförmigen Körpers orientiert angeordnet sein kann.

Die erfindungsgemäße Vorrichtung wird im Folgenden anhand bevorzugter Ausführungsformen erläutert, die in den Figuren der Zeichnung dargestellt sind. Darin zeigen:
- Fig. 1: eine erste bevorzugte Ausführungsform der erfindungsgemäßen Vorrichtung in einer schematischen Querschnittsansicht;
- Fig. 2: eine zweite bevorzugte Ausführungsform der erfindungsgemäßen Vorrichtung in einer schematischen Querschnittsansicht.

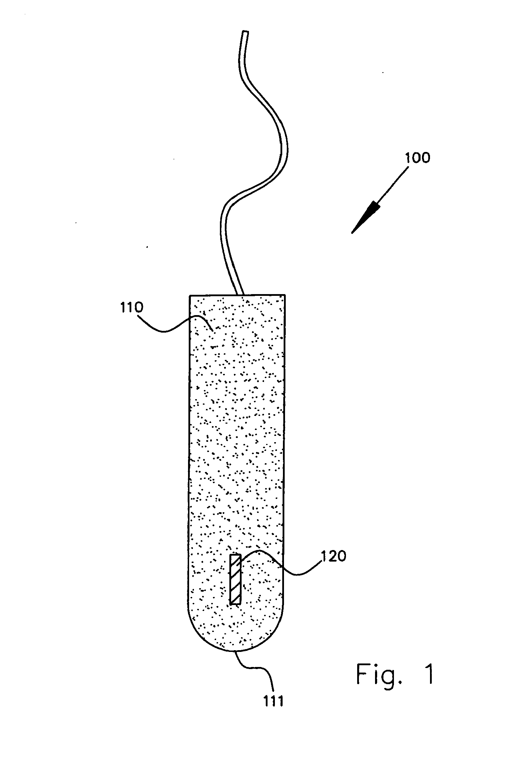

Die in Figur 1 dargestellte erfindungsgemäße Tampon-Vorrichtung 100 zum Einführen in die weibliche Vulva enthält eine Emballage 110 aus saugfähiger Substanz zur Aufnahme von Menstruationsflüssigkeit, wobei in die Emballage 110 eine mit dem Erdmagnetfeld in Wechselwirkung tretende magnetische Substanz 120 integriert ist. Die magnetische Substanz 120 ist dabei von Eisen gebildet und in Form eines Stabmagneten ausgebildet, der im Inneren der Emballage 110 angeordnet ist.

Die Emballage 110 ist im Wesentlichen in Form eines zylinderförmigen Körpers mit abgerundeten Stirnflächen 111 ausgebildet, wobei der Stabmagnet im Bereich der Längsachse des zylinderförmigen Körpers vorgesehen und in Richtung der Längsachse des zylinderförmigen Körpers orientiert ist.

Der zylinderförmige Körper weist eine Länge von etwa 3,0 cm bei einem Durchmesser von etwa 0,7 cm auf. Der Stabmagnet weist eine Länge von etwa 3 mm bei einem Durchmesser von etwa 2 mm auf.

Das von dem Stabmagneten verursachte Magnetfeld weist eine Stärke auf, die etwa dem 2-fachen der Stärke des Erdmagnetfeldes entspricht.

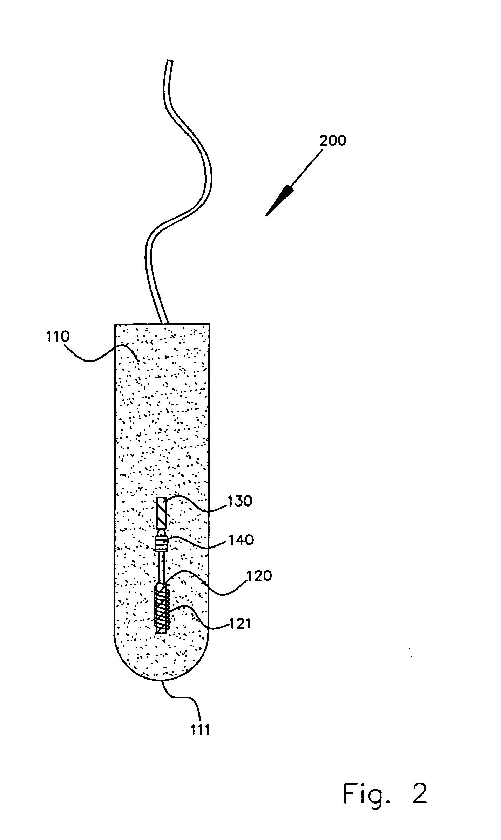

Die in Figur 2 dargestellte erfindungsgemäße Tampon-Vorrichtung 200 zum Einführen in die weibliche Vulva ist prinzipiell ähnlich wie die in Figur 1 dargestellte Tampon-Vorrichtung 100 aufgebaut und enthält eine Emballage 110 aus saugfähiger Substanz zur Aufnahme von Menstruationsflüssigkeit, wobei in die Emballage 110 eine Stromquelle 130, eine von einer elektrischen Spule 121 umhüllte magnetische Substanz 120 sowie eine Aktivierungseinrichtung 140 zum Steuern des die Spule 121 durchfließenden elektrischen Stroms integriert ist. Die Aktivierungseinrichtung 140 erzeugt dabei eine Rechteckspannung, die in der Spule 121 ein entsprechendes magnetisches Wechselfeld aufbaut.

Die Emballage 110 ist auch hier im Wesentlichen in Form eines zylinderförmigen Körpers mit abgerundeten Stirnflächen 111 ausgebildet, wobei die Stromquelle 130, die die magnetische Substanz 120 umhüllende elektrische Spule 121 sowie die Aktivierungseinrichtung 140 im Bereich der Längsachse des zylinderförmigen Körpers vorgesehen sind.

Die oben erläuterten Ausführungsbeispiele der Erfindung dienen lediglich dem Zweck eines besseren Verständnisses der durch die Ansprüche definierten erfindungsgemäßen Lehre, die als solche durch das Ausführungsbeispiel nicht eingeschränkt ist.

## Patentansprüche

1. Tampon-Vorrichtung (100) zum Einführen in die weibliche Vulva, mit einer Emballage (110) aus saugfähiger Substanz zur Aufnahme von Menstruationsflüssigkeit, wobei in die Emballage (110) eine mit dem Erdmagnetfeld in Wechselwirkung tretende magnetische Substanz (120) integriert ist, **dadurch gekennzeichnet, dass** die magnetische Substanz (120) in Form eines Stabmagneten ausgebildet ist, der in Richtung der Längsachse des zylinderförmigen Körpers orientiert angeordnet ist, der zylinderförmige Körper eine Länge im Bereich von etwa 2,0 cm bis 5,0 cm bei einem Durchmesser von etwa 0,5 cm bis 1,5 cm aufweist, und ein mittels der magnetischen Substanz (120) verursachtes Magnetfeld eine Stärke aufweist, die dem 0,5-fachen bis 2-fachen der Stärke des Erdmagnetfeldes entspricht.

2. Vorrichtung nach Anspruch 1, **dadurch gekennzeichnet, dass** die magnetische Substanz (120) von Eisen gebildet ist.

3. Vorrichtung nach Anspruch 1, **dadurch gekennzeichnet, dass** die magnetische Substanz (120) von Nickel gebildet ist.

4. Vorrichtung nach einem der vorhergehenden Ansprüche, **dadurch gekennzeichnet, dass** die magnetische Substanz (120) im Inneren der Emballage (110) angeordnet ist.

5. Vorrichtung nach einem der vorhergehenden Ansprüche, **dadurch gekennzeichnet, dass** die Emballage (110) im wesentlichen im Form eines zylinderförmigen Körpers mit abgerundeten Stirnflächen (111) ausgebildet ist, wobei die magnetische Substanz (120) im Bereich der Längsachse des zylinderförmigen Körpers vorgesehen ist.

6. Vorrichtung nach einem der vorhergehenden Ansprüche, **dadurch gekennzeichnet, dass** der Stabmagnet eine Länge im Bereich von 0,5 mm bis 10 mm bei einem Durchmesser von 0,5 mm bis 5 mm aufweist.

7. Tampon-Vorrichtung (100) zum Einführen in die weibliche Vulva, mit einer Emballage (110) aus saugfähiger Substanz zur Aufnahme von Menstruationsflüssigkeit, wobei in die Emballage (110) eine mit dem Erdmagnetfeld in Wechselwirkung tretende magnetische Substanz (120) integriert ist, wobei in die Emballage (110) eine Stromquelle (130), eine von einer elektrischen Spule (121) umhüllte magnetische Substanz (120) sowie eine Aktivierungseinrichtung (140) zum Steuern des die Spule (121) durchfließenden elektrischen Stroms integriert ist, **dadurch gekennzeichnet, dass** die Emballage (110) im wesentlichen im Form eines zylinderförmigen Körpers mit abgerundeten Stirnflächen (111) ausgebildet ist, wobei die Stromquelle (130), die magnetische Substanz (120) umhüllende elektrische Spule (121) sowie die Aktivierungseinrichtung (140) im Bereich der Längsachse des zylinderförmigen Körpers vorgesehen sind, und der zylinderförmige Körper dabei eine Länge im Bereich von etwa 2,0 cm bis 5,0 cm bei einem Durchmesser von etwa 0,5 cm bis 1,5 cm aufweist.

8. Vorrichtung nach Anspruch 7, **dadurch gekennzeichnet, dass** die Aktivierungseinrichtung (140) eine Rechteckspannung erzeugt.

9. Vorrichtung nach Anspruch 7, **dadurch gekennzeichnet, dass** die Aktivierungseinrichtung (140) einen sinusförmigen Spannungsverlauf erzeugt.

10. Vorrichtung nach einem der Ansprüche 7 bis 9, **dadurch gekennzeichnet, dass** die magnetische Substanz (120) von Eisen gebildet ist.

11. Vorrichtung nach einem der Ansprüche 7 bis 10, **dadurch gekennzeichnet, dass** die magnetische Substanz (120) in Form eines innerhalb der Spule (121) angeordneten Stabmagneten ausgebildet ist.

12. Vorrichtung nach einem der Ansprüche 7 bis 11, **dadurch gekennzeichnet, dass** die Stromquelle (130), die die magnetische Substanz (120) umhüllende elektrische Spule (121) sowie die Aktivierungseinrichtung (140) im Inneren der Emballage (110) angeordnet sind.

13. Vorrichtung nach einem der Ansprüche 7 bis 12, **dadurch gekennzeichnet, dass** die magnetische Substanz (120) in Form eines Stabmagneten ausgebildet ist, der in Richtung der Längsachse des zylinderförmigen Körpers orientiert angeordnet ist.

## Claims

1. A tampon device (100) for introduction into the female vulva, comprising a packing material (110) of an absorbent material for taking up menstrual fluid, wherein said packing material (110) surrounds a magnetic substance (120) that interacts with the earth's magnetic field, **characterized in that** the magnetic substance (120) is in the form of a bar magnet, oriented in the direction of the longitudinal axis of the cylindrical body, that said cylindrical body has a length ranging from approximately 2.0 cm to 5.0 cm and a diameter ranging from approximately 0.5 cm to 1.5 cm, and that a magnetic field produced by means of said magnetic substance (120) has a strength that is equal to from 0.5 times to 2 times the strength of the earth's magnetic field.

2. The device according to claim 1, **characterized in that** said magnetic substance (120) is of iron.

3. The device according to claim 1, **characterized in that** said magnetic substance (120) is of nickel.

4. The device according to any one of the previous claims, **characterized in that** said magnetic substance (120) is disposed in the interior of said packing material (110).

5. The device according to any one of the previous claims, **characterized in that** said packing material (110) is substantially in the form of a cylindrical body with rounded end faces (111) and the magnetic substance (120) is disposed in the region of the longitudinal axis of the cylindrical body.

6. The device according to any one of the previous claims, **characterized in that** said bar magnet has a length ranging from 0.5 mm to 10 mm and a diameter of from 0.5 mm to 5 mm.

7. A tampon device (100) for introduction into the female vulva, comprising a packing material (110) of an absorbent material for taking up menstrual fluid, wherein said packing material (110) surrounds a magnetic substance (120) which interacts with the earth's magnetic field, and said packing material (110) encompasses a power source (130), a magnetic substance (120) surrounded by an electric coil (121), and activating means (140) for controlling the electric current flowing through said coil (121), **characterized in that** said packing material (110) is substantially in the form of a cylindrical body with rounded end faces (111), and said power source (130), said coil (121) surrounding said magnetic substance (120) and also said activating means (140) are provided in the region of the longitudinal axis of said cylindrical body, and said cylindrical body has a length ranging from approximately 2.0 cm to 5.0 cm and a diameter of from approximately 0.5 cm to 1.5 cm.

8. The device according to claim 7, **characterized in that** said activating means (140) produces a square wave voltage.

9. The device according to claim 7, **characterized in that** said activating means (140) produces a sinusoidal voltage curve.

10. The device according to any one of claims 7 to 9, **characterized in that** said magnetic substance (120) is of iron.

11. The device according to any one of claims 7 to 10, **characterized in that** said magnetic substance (120) is in the form of a bar magnet disposed within said coil (121).

12. The device according to any one of claims 7 to 11, **characterized in that** said power source (130), said coil (121) surrounding said magnetic substance (120) and also said activating means (140) are disposed in the interior of said packing material (110).

13. The device according to any one of claims 7 to 12, **characterized in that** said magnetic substance (120) is in the form of a bar magnet, oriented in the direction of the longitudinal axis of the cylindrical body.

## Revendications

1. Dispositif de tampon (100) destiné à être introduit dans la vulve féminine, comprenant un emballage (110) en substance absorbante pour l'absorption de liquide menstruel, une substance magnétique (120) entrant en interaction avec le champ magnétique terrestre étant intégrée dans l'emballage (110), **caractérisé en ce que** la substance magnétique (120) est réalisée sous forme d'un aimant en forme de bâtonnet qui est agencé de manière orientée en direction de l'axe longitudinal du corps cylindrique, le corps cylindrique présente une longueur de l'ordre d'environ 2,0 cm à 5,0 cm pour un diamètre d'environ 0,5 cm à 1,5 cm, et un champ magnétique engendré au moyen de la substance magnétique (120) présente une intensité qui correspond à entre 0,5 fois et 2 fois l'intensité du champ magnétique terrestre.

2. Dispositif selon la revendication 1, **caractérisé en ce que** la substance magnétique (120) est constituée de fer.

3. Dispositif selon la revendication 1, **caractérisé en ce que** la substance magnétique (120) est constituée de nickel.

4. Dispositif selon une des revendications précédentes, **caractérisé en ce que** la substance magnétique (120) est agencée à l'intérieur de l'emballage (110).

5. Dispositif selon une des revendications précédentes, **caractérisé en ce que** l'emballage (110) est essentiellement réalisé sous forme d'un corps cylindrique dont les surfaces frontales (111) sont arrondies, la substance magnétique (120) étant prévue dans la zone de l'axe longitudinal du corps cylindrique.

6. Dispositif selon une des revendications précédentes, **caractérisé en ce que** l'aimant en forme de bâtonnet présente une longueur de l'ordre de 0,5 mm à 10 mm pour un diamètre de 0,5 mm à 5 mm.

7. Dispositif de tampon (100) destiné à être introduit dans la vulve féminine, comprenant un emballage (110) en substance absorbante pour l'absorption de liquide menstruel, une substance magnétique (120) entrant en interaction avec le champ magnétique terrestre étant intégrée dans l'emballage (110), une source de courant (130), une substance magnétique (120) enveloppée d'une bobine électrique (121) ainsi qu'un appareillage d'activation (140) pour le pilotage du courant électrique traversant la bobine (121) étant intégrés dans l'emballage (110), **caractérisé en ce que** l'emballage (110) est essentiellement réalisé sous forme d'un corps cylindrique dont les surfaces frontales (111) sont arrondies, la source de courant (130), la bobine électrique (121) enveloppant la substance magnétique (120) ainsi que l'appareillage d'activation (140) étant prévus dans la zone de l'axe longitudinal du corps cylindrique, et le corps cylindrique présentant ici une longueur de l'ordre d'environ 2,0 cm à 5,0 cm pour un diamètre d'environ 0,5 cm à 1,5 cm.

8. Dispositif selon la revendication 7, **caractérisé en ce que** l'appareillage d'activation (140) génère une tension carrée.

9. Dispositif selon la revendication 7, **caractérisé en ce que** l'appareillage d'activation (140) génère une courbe de tension sinusoïdale.

10. Dispositif selon une des revendications de 7 à 9, **caractérisé en ce que** la substance magnétique (120) est constituée de fer.

11. Dispositif selon une des revendications de 7 à 10, **caractérisé en ce que** la substance magnétique (120) est réalisée sous forme d'un aimant en forme de bâtonnet qui est agencé à l'intérieur de la bobine (121).

12. Dispositif selon une des revendications de 7 à 11, **caractérisé en ce que** la source de courant (130), la bobine électrique (121) enveloppant la substance magnétique (120) ainsi que l'appareillage d'activation (140) sont agencés à l'intérieur de l'emballage (110).

13. Dispositif selon une des revendications de 7 à 12, **caractérisé en ce que** la substance magnétique (120) est réalisée sous forme d'un aimant en forme de bâtonnet qui est agencé de manière orientée en direction de l'axe longitudinal du corps cylindrique.
